(19)

Europäisches Patentamt
European Patent Office
Office européen des brevets

(11) **EP 4 447 068 A1**

(12) **EUROPEAN PATENT APPLICATION**

(43) Date of publication:
**16.10.2024 Bulletin 2024/42**

(21) Application number: **24170013.7**

(22) Date of filing: **12.04.2024**

(51) International Patent Classification (IPC):
**G16H 50/30** (2018.01)        **A61B 5/029** (2006.01)
**A61B 5/00** (2006.01)

(52) Cooperative Patent Classification (CPC):
**G16H 50/30; A61B 5/029; A61B 5/7275**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.04.2023   IN 202321017923**

(71) Applicant: **Tata Consultancy Services Limited Maharashtra (IN)**

(72) Inventors:
• **BHATTACHARYA, SAKYAJIT**
  **700135 Kolkata (IN)**

• **ROY, DIBYENDU**
  **700160 Kolkata (IN)**
• **SINHA, ANIRUDDHA**
  **700 091 Kolkata (IN)**
• **GHOSE, AVIK**
  **700 091 Kolkata (IN)**
• **SHARMA, VARSHA**
  **700160 Kolkata (IN)**
• **MAZUMDER, OISHEE**
  **700160 Kolkata (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD AND SYSTEM FOR DETERMINING POST-EXERCISE RECOVERY SCORE USING PERSONALIZED CARDIAC MODEL**

(57)    It is important to monitor the cardiac condition of an individual, outside the clinic, using wearable physiological sensors. However, existing methods for calculating the cardiac risk score of an individual are primarily based on static information like individual's metadata, lifestyle, family history, clinical assessment, etc. but do not consider the cardiac state in a daily living scenario using wearable-based measurements. Embodiments herein provide a method and a system for determining post-exercise cardiac score in a recovery period using personalized cardiac model. A clinical decision support system (CDSS) is disclosed to predict cardiac recovery score of a subject in post-exercise conditions. The system employs a hybrid approach using a computational cardiac model and wearable data. Further, several personalized cardiac parameters are simulated using a cardiovascular simulation (CVS) platform. These parameters are used along with the wearable ECG data and meta-data information to derive the post-exercise recovery score.

FIG. 3

**EP 4 447 068 A1**

**Description**

CROSS-REFERENCE TO RELATED APPLICATIONS AND PRIORITY

The present application claims priority from Indian patent application number 202321017923, filed on April 15, 2023.

TECHNICAL FIELD

The disclosure herein generally relates to the field of clinician decision support systems and more particularly, to a method and system for determining post-exercise cardiac score in a recovery period using personalized cardiac model.

BACKGROUND

Cardiovascular diseases (CVDs), which are the world's leading cause of mortality and serious illness, primarily impact the circulatory system of humans. As a result, healthcare expenditures are significantly affected. Sudden cardiac death (SCD) and coronary artery disorders account for about 50% of cardiac deaths. Moreover, in many of these cases, SCD occurs in people with no history of CVDs and only a few of the usual cardiac risk factors.

The risk of sudden cardiac arrest is higher in obese patients with high body-metabolic index (BMI). Moreover, there could be underlying pathology that could increase the cardiac risk of normal people during occasional exercises or exertions and is unnoticed without any cardiac symptoms in daily living conditions. Hence, stress tests are frequently advised by doctors to detect cardiac risk factors so that the heart's response to stress could be taken into consideration and evaluated effectively. Even for trained athletes, the stress test is a common practice, most of the time, they over-exert their cardiac systems, not knowing where to stop, resulting in sudden cardiac arrest. Therefore, it is important to monitor the cardiac condition of an individual, outside the clinic, using wearable physiological sensors. The dynamics of recovery for an individual to the resting state, immediately after exercise, provides a good indication of cardiac condition.

SUMMARY

Embodiments of the disclosure present technological improvements as solutions to one or more of the above-mentioned technical problems recognized by the inventors in conventional systems. For example, in one embodiment, a method and system for determining post-exercise cardiac score in a recovery period using personalized cardiac model is provided.

In one aspect, a processor-implemented method for determining post-exercise cardiac score in a recovery period using personalized cardiac model is provided. The processor-implemented method comprising receiving, via an input/output interface, a meta-data information of a subject, wherein the meta-data of the subject comprising height, weight, and age of the subject. Further, the processor-implemented method includes collecting wearable electrocardiogram (ECG) data of the subject. Herein, the subject is being instructed to complete a predefined physical activity for a predefined period, sit up straight after completing the predefined physical activity for a predefined period to collect data, and remain stable with no movement during the data collection.

Furthermore, the processor-implemented method includes estimating one or more cardiac parameters in each cardiac cycle of the wearable ECG data associated with the subject using a cardiovascular simulation (CVS) model. Herein, the one or more cardiac parameters include an ejection fraction (EF), an arterial blood pressure (BP), cardiac output (CO) and a continuous heart rate (HR). Further, the processor-implemented method includes calculating a body-metabolic index (BMI) of the subject using a standard scoring method and a normal BMI range. Furthermore, the processor-implemented method includes estimating a meta score by combining the received meta-data information, the estimated one or more cardiac parameters in each cardiac cycle of the wearable ECG data and the calculated BMI of the subject. Finally, the method includes determining the cardiac score in a recovery period post-exercise from the estimated meta score based on a predefined normalized scale.

In another aspect, a system for determining post-exercise cardiac score in a recovery period using personalized cardiac model is provided. The system includes at least one memory storing programmed instructions, one or more Input /Output (I/O) interfaces, and one or more hardware processors operatively coupled to the at least one memory, wherein the one or more hardware processors are configured by the programmed instructions to receive a meta-data information of a subject, wherein the meta-data of the subject comprising height, weight, and age of the subject. Further, the one or more hardware processors are configured by the programmed instructions to collect wearable electrocardiogram (ECG) data of the subject. Herein, the subject is being instructed to complete a predefined physical activity for a predefined period, sit up straight after completing the predefined physical activity for a predefined period to collect data, and remain stable with no movement during the data collection.

Furthermore, the one or more hardware processors are configured by the programmed instructions to estimating one or more cardiac parameters in each cardiac cycle of the wearable ECG data associated with the subject using a cardiovascular simulation (CVS) model. Herein, the one or more cardiac parameters include an ejection fraction (EF), an arterial blood pressure (BP), cardiac output (CO) and a continuous heart rate (HR). Furthermore, the one or more hardware processors are configured by the programmed instructions to calculating a body-metabolic index (BMI) of the subject using a standard scoring method and a normal BMI range. Furthermore, the one or more hardware processors are configured by the programmed instructions to estimating a meta score by combining the received meta-data information, the estimated one or more cardiac parameters in each cardiac cycle of the wearable ECG data and the calculated BMI of the subject. Finally, the one or more hardware processors are configured by the programmed instructions to determining the cardiac score in a recovery period post-exercise from the estimated meta score based on a predefined normalized scale.

In yet another aspect, one or more non-transitory machine-readable information storage mediums are provided comprising one or more instructions, which when executed by one or more hardware processors causes a method for determining post-exercise cardiac score in a recovery period using personalized cardiac model. The processor-implemented method comprises receiving, via an input/output interface, a meta-data information of a subject, wherein the meta-data of the subject comprises height, weight, and age of the subject. Further, the processor-implemented method includes collecting wearable electrocardiogram (ECG) data of the subject. Herein, the subject is being instructed to complete a predefined physical activity for a predefined period, sit up straight after completing the predefined physical activity for a predefined period to collect data, and remain stable with no movement during the data collection.

Furthermore, the processor-implemented method includes estimating one or more cardiac parameters in each cardiac cycle of the wearable ECG data associated with the subject using a cardiovascular simulation (CVS) model. Herein, the one or more cardiac parameters include an ejection fraction (EF), an arterial blood pressure (BP), cardiac output (CO) and a continuous heart rate (HR). Further, the processor-implemented method includes calculating a body-metabolic index (BMI) of the subject using a standard scoring method and a normal BMI range. Furthermore, the processor-implemented method includes estimating a meta score by combining the received meta-data information, the estimated one or more cardiac parameters in each cardiac cycle of the wearable ECG data and the calculated BMI of the subject. Finally, the method includes determining the cardiac score in a recovery period post-exercise from the estimated meta score based on a predefined normalized scale.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates a network diagram of a system for determining post-exercise cardiac score in a recovery period using personalized cardiac model, in accordance with some embodiments of the present disclosure.
FIG. 2 is an exemplary flow diagram illustrating a method for determining post-exercise cardiac score in a recovery period using personalized cardiac model, in accordance with some embodiments of the present disclosure.
FIG. 3 is a block diagram to illustrate a cardiovascular simulation (CVS) model generating simulated cardiac parameters, in accordance with some embodiments of the present disclosure.
FIGS. 4A and 4B (collectively referred as FIG. 4) are schematic diagrams to show weight versus end evaluated cardiac score plot with model outputs (FIG. 4A) and without model outputs (FIG. 4B), in accordance with some embodiments of the present disclosure.

DETAILED DESCRIPTION OF EMBODIMENTS

Exemplary embodiments are described with reference to the accompanying drawings. In the figures, the leftmost digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The risk of sudden cardiac arrest is higher in obese patients with high body-metabolic index (BMI). Moreover, there could be underlying pathology that could increase the cardiac risk of normal people during occasional exercises or exertions and is unnoticed without any cardiac symptoms in daily living conditions. Hence, stress tests are frequently advised by doctors to detect cardiac risk factors so that the heart's response to stress could be taken into consideration and evaluated effectively. Even for trained athletes, the stress test is a common practice. However, most of the time, they over-exert their cardiac systems, not knowing where to stop and hence suffering from sudden cardiac arrest. Therefore, it is important to monitor the cardiac condition of an individual, outside the clinic, using wearable physiological sensors. The dynamics of recovery for an individual to the resting state, immediately after exercise, provides a good indication of cardiac condition. However, there are a few standard methods for calculating the cardiac risk score of an individual. These are primarily based on static information like individual's metadata, lifestyle, family history, clinical assessment, etc. but do not consider the cardiac state in a daily living scenario using wearable-based measurements.

Therefore, embodiments herein provide a method and system for determining post-exercise cardiac score in a recovery period using personalized cardiac model. Initially, the system evaluates scores based on the meta-data of the subject and extracts features from a recorded electrocardiogram (ECG) data (after exercise). Then, a CVS model is used to generate several cardiac parameters, including an ejection fraction (EF), an arterial blood pressure (BP), etc. After that, using those parameters and the previously evaluated features, the system determines post-exercise cardiac score. It would be appreciated that the model-based cardiac scores better correlate with the weight of the subjects, so, it could be utilized as a clinical decision support system for physicians.

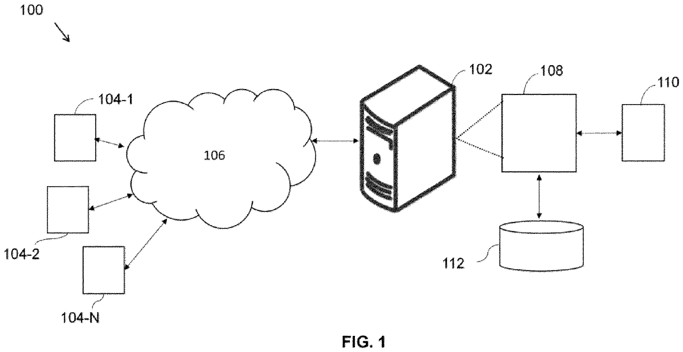

Referring now to the drawings, and more particularly to FIG. 1 through FIG. 4, where similar reference characters denote corresponding features consistently throughout the figures, there are shown preferred embodiments and these embodiments are described in the context of the following exemplary system and/or method.

FIG. 1 illustrates a network diagram of a system 100 for determining post-exercise cardiac score in a recovery period using personalized cardiac model. Although the present disclosure is explained considering that the system 100 is implemented on a server, it may also be present elsewhere such as a local machine. It may be understood that the system 100 comprises one or more computing devices 102, such as a laptop computer, a desktop computer, a notebook, a workstation, a cloud-based computing environment and the like. It will be understood that the system 100 may be accessed through one or more input/output interfaces 104-1, 104-2... 104-N, collectively referred to as I/O interface 104. Examples of the I/O interface 104 may include, but are not limited to, a user interface, a portable computer, a personal digital assistant, a handheld device, a smartphone, a tablet computer, a workstation and the like. The I/O interface 104 are communicatively coupled to the system 100 through a network 106.

In an embodiment, the network 106 may be a wireless or a wired network, or a combination thereof. In an example, the network 106 can be implemented as a computer network, as one of the different types of networks, such as virtual private network (VPN), intranet, local area network (LAN), wide area network (WAN), the internet, and such. The network 106 may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network 106 may include a variety of network devices, including routers, bridges, servers, computing devices, storage devices. The network devices within the network 106 may interact with the system 100 through communication links.

The system 100 may be implemented in a workstation, a mainframe computer, a server, and a network server. In an embodiment, the computing device 102 further comprises one or more hardware processors 108, one or more memory 110, hereinafter referred as a memory 110 and a data repository 112, for example, a repository 112. The data repository 112 may also be referred as a dynamic knowledge base 112 or a knowledge base 112. The data repository 112 may include a plurality of abstracted piece of code for refinement and data that is processed, received, or generated as a result of the execution of the plurality of modules in the module(s). Although the data repository 112 is shown external to the system 100, it will be noted that, in alternate embodiments, the data repository 112 can also be implemented internal to the system 100, and communicatively coupled to the system 100. The data contained within such external database may be periodically updated. For example, new data may be added into the database and/or existing data may be modified and/or non-useful data may be deleted from the database.

The memory 110 is in communication with the one or more hardware processors 108, wherein the one or more hardware processors 108 are configured to execute programmed instructions stored in the memory 110, to perform various functions as explained in the later part of the disclosure. The repository 112 may store data processed, received, and generated by the system 100. The memory 110 further comprises a plurality of modules (not shown). Further, the plurality of modules can be used by hardware, by computer-readable instructions executed by the one or more hardware processors 108, or by a combination thereof.

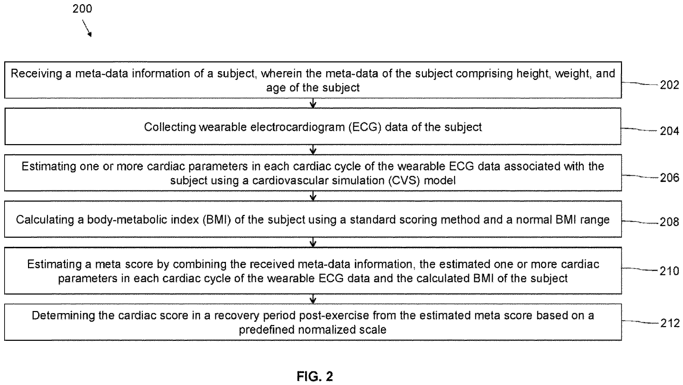

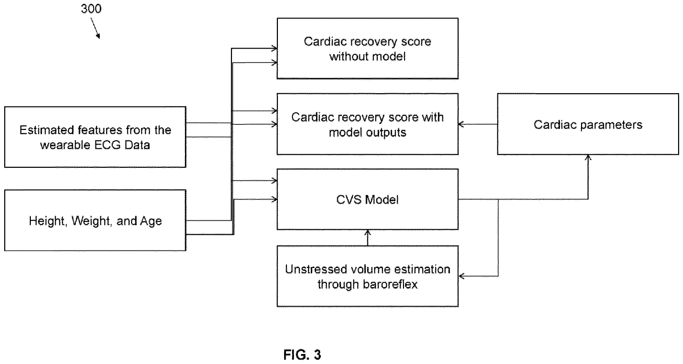

The system 100 supports various connectivity options such as BLUETOOTH®, USB, ZigBee and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. The components and functionalities of the system 100 are described further in detail. Functions of the components of the system 100 are now explained with reference to FIG. 3 through steps of flow diagram in FIG. 2.

FIG. 2 is an exemplary flow diagrams illustrating a processor-implemented method 200 for determining post-exercise cardiac score in a recovery period using personalized cardiac model implemented by the system of FIG. 1, according to some embodiments of the present disclosure. A clinical decision support system (CDSS) scheme is disclosed to predict cardiac recovery score of a subject in post-exercise conditions, employing a hybrid approach using a computational cardiac model and wearable data. Further, several personalized cardiac parameters are simulated using a cardiovascular simulation platform. These parameters, when used along with the wearable electrocardiogram (ECG) data and meta-data information to derive the post-exercise recovery score, demonstrate an improvement in capturing the stochastic nature of underlying cardiac conditions among individuals under stress.

Initially at step 202 of the method 200, a meta-data information of a subject is received, via an input/output interface 104, as an input. The meta-data of the subject comprising height, weight, and age of the subject.

At the next step 204 of the method 200, the one or more hardware processors 108 are configured by the programmed instructions to collect wearable ECG data of the subject. Herein, the subject is being instructed to complete a predefined physical activity for a predefined period, sit up straight after completing the predefined physical activity for a predefined period to collect data, and remain stable with no movement during the data collection.

In one illustration, to collect the data of a subject, he/she is requested to run on a treadmill for 3 minute at 8 kilometer/hour speed. After completing the activity, the participant is required to sit up straight so that the data collection may begin. To reduce the effect of motion artifacts, the subject is instructed to remain stable with no movement during data collection. The whole process consumes around 4-5 min per subject. In the end, the meta-data information (such as age, height, and weight) is also noted. Additionally, several reference BPs have been measured during data recording for each subject. The entire dataset contains the post-exercise wearable ECG data, which is used to generate the cardiac recovery score.

At the next step 206 of the method 200, the one or more hardware processors 108 are configured by the programmed instructions to estimate one or more cardiac parameters in each cardiac cycle of the wearable ECG data associated with the subject using a cardiovascular simulation (CVS) model. Herein, the one or more cardiac parameters include an ejection fraction (EF), an arterial pressure (BP), a cardiac output (CO) and a continuous heart rate (HR) as shown in FIG. 3.

In one embodiment, the CVS model comprising a pair of atriums and ventricles functioning as a pulsatile pump. The left atrium ventricle pair creates the systemic circular path to pump oxygenated blood to all body tissues via the aorta, and the right atrium-ventricle pair drives the deoxygenated blood to the lungs forming the pulmonic circulation. The rhythmic unidirectional blood flows across the heart chambers are controlled by the synchronized opening and closing of the four cardiac valves. The following variables of the CVS model are estimated from the captured ECG data and the meta-data information of a subject.

In another embodiment, the continuous heart rate (HR) and one or more cardiac compliance parameters are estimated using the wearable ECG data. Based on this information, the pressures-volumes are updated across the cardiac chambers based on the estimated continuous heart rate and one or more cardiac compliance parameters. To determine the HR from the captured wearable ECG data, R-to-R durations are determined in each cardiac cycle.

**First and last heart rate readings:** The first and last HR readings of the post-activity state reflects how much the elevated HR is and how much the HR is at rest. This is related to age and scores are assigned likewise, 0 in the normal range and increasing score as the HR goes away from the normal range as determined by the American heart association.

**Peak and trough height:** The peaks are gradually decreasing toward a normal level, ideally following an exponential distribution. This is because the decrease is sharp in the initial stages and then normalizes at the later stage, gradually going to 0 where the HR stabilizes. An exponential pdf $\lambda e^{-\lambda}$ is fitted to the peaks and estimate $\lambda$ as $\lambda$. Further, calculating the distance between the empirical distribution function and the reference distribution function using a Kolmogorov-Smirnov test (KS-test). The calculated distance between the empirical distribution function and the reference distribution function tells whether the samples belong to the reference distribution. Furthermore, using the *'dgof* package in R, to assign a score of 0 if the test shows the samples belong to the exponential distribution, and a score 1 otherwise. The procedure is the same for the trough heights.

**When peak $\leq$ 100 for the first time** - Ideally, by 120 seconds post-activity, HR becomes normal, i.e., in the range [60, 100]. So, an ideal scoring technique assigns 0 if the time ($t_N$) is less than 120 and gradually increases when $t_N$ is more than 120. Thus, a straight line from 120 to the highest time point such that its value is 0 at or less than 120, 1 for 180 can indicate the scoring technique.

**Sudden spikes in peaks and troughs** - A sudden spike means a sharp increase or decrease (more than 10% change of the previous value) in peaks or troughs. These can happen more frequently just after the activity as there is noise in HR and becomes rarer as time goes on. Hence, the distribution of the spikes can be fitted to a Poisson density:

$$P(X = k) = \frac{\lambda^k e^{-\lambda}}{k!} \; for \; k = 0,1,2.. \tag{1}$$

wherein the probability of more spikes ideally gets less. The mean number of spikes $\lambda$ is estimated from the observed values. The KS-test is conducted and 0 and 1 is assigned accordingly as the null hypothesis is accepted or rejected. A similar analysis has been performed for troughs going below 60.

**Trend** - A trend pattern in a time series exists when there is a long-term change in the mean level. Let $X_t$ be original data, Then the de-trended data is:

$$Y_t = X_t - T_t,$$

wherein $T_t$ is the trend component and $X_t^* = \frac{X_t^\lambda - 1}{\lambda}$ , $\lambda \neq 0$ and the transformation parameter $\lambda$ can be chosen by the Box-Cox method.

Similarly, the data can be de-seasonalized as $Z_t = X_t - S_t$ where the seasonal effect $S_t$ can be found via Box-Cox transformation. This transformation also gives us the de-trended and de-seasonalized *component Xt* = $X_t$ -$T_t$-$S_t$.

Then a suitable measure of trend is $\hat{T}_t = \frac{1 - Var\,(Xt)}{Var\,(Zt)}$ , Thus $0 \leq \hat{T}_t \leq 1$, and ideal situation is that there is no long-term change in HR, i.e., the decreasing rate pattern is homogeneous. We thus assign a score of 0 if the estimated trend does not cross 0.2, 1 if it lies in [0.2, 0.5], and 2 otherwise.

The meta-data information such as height and weight are utilized to define a total blood volume and an unstressed blood volume of the subject. Total blood volume is fixed during the simulation, whereas the unstressed blood volume of the subject is auto regulated by a baroreflex autoregulation principle. Based on the pressure-volume dynamics of the model, several indicators of the heard such as EF, BP, CO, etc. to assess the cardiac condition of the subject. These are already validated with parameters for healthy and disease conditions including valvular and ischemic diseases.

Referring back to FIG. 2, at the step 208 of the method 200, the one or more hardware processors 108 are configured by the programmed instructions to calculate a body-metabolic index (BMI) of the subject using a standard scoring method and a normal BMI range.

At the next step 210 of the method 200, the one or more hardware processors 108 are configured by the programmed instructions to estimate a meta score by combining the received meta-data information, the estimated one or more cardiac parameters in each cardiac cycle of the wearable ECG data and the calculated BMI of the subject. A high meta score means wellness, and a low score indicates disease, and the normalized scale is between 0 to 10. Hence, the final score would be:

$$S = \frac{(P - M)}{P \; X 10}$$

wherein the values of *P are* 20 and 40 for without and with model conditions respectively.

Finally, at the last step 212 of the method 200, the one or more hardware processors 108 are configured by the programmed instructions to determine the cardiac score in a recovery period post-exercise from the estimated meta score based on a predefined normalized scale.

## Experiment:

A whole wearable ECG data window is divided into 20-sec intervals to calculate a subject's cardiac scores. Similarly, a single score is generated for each of EF and BP from the model for each 20-sec time window. Then, cardiac scores are generated in each of the 20-sec windows with and without the model outputs. Comparing the scores with and without the model, it is seen that both scores are approaching a steady-state value at the end of the data collection step. However, based on the initially evaluated scores, the score with the model is more rectified than the without model condition, demonstrating the efficacy of the cardiac score predictor of the model outputs.

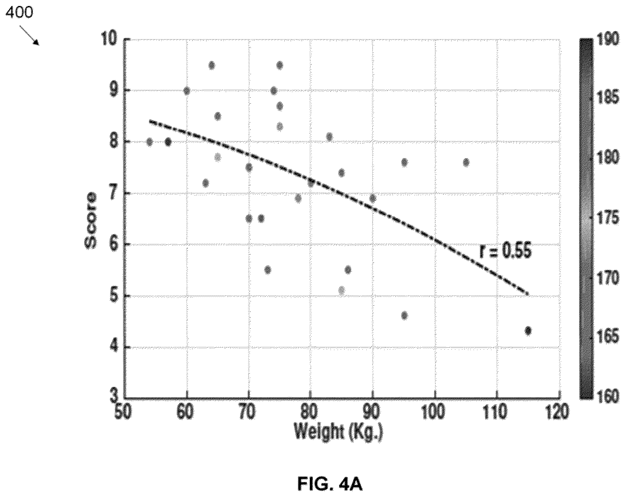

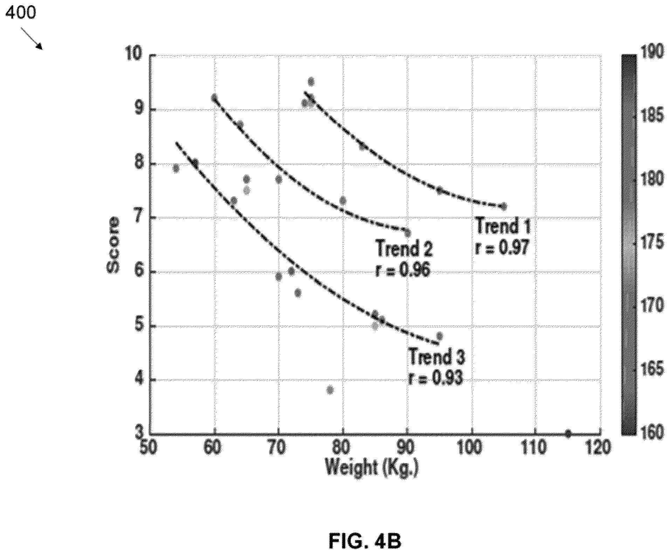

FIGS. 4A, and 4B, schematic diagrams illustrating a plot of weight versus end cardiac scores for all the subjects with and without model outputs respectively. It can be seen from Fig. 4B, the evaluated scores using the wearable data and the CVS model have a better negative correlation (r > 0.93) with the weight than compared to the scores derived using only the wearable data (Fig. 4A). Additionally, from Fig. 4B it is observed that there are groups of subjects (reflected in the scores) for whom separate correlations (three in this case) with the weight could be generated. This indicates that by using the model data, individuals can be further segregated who are having different weight score relationships.

In another aspect, first evaluate a BMI surface considering the maximum and minimum ranges of the height and weight data. Then, the normal distance of scores is computed from the BMI plane. From the outcomes, it is seen that though the mean absolute distances (MAD) are similar, the standard deviation (STD) of the distance are greater with the model outputs than without model. Thus, it is inferred that the model-associated cardiac recovery score is able to better capture the variations among the subjects compared to static BMI information.

The written description describes the subj ect matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address the problem of generated molecules from the knocked-out gene expression profiles which are significantly unrelated (having low Tanimoto coefficients) with the known inhibitors of the genes. Embodiments herein provide a method and a system for a cell specific model where gene expressions are processed via a pretrained SMILES variational autoencoder to produce new molecules. The initially evaluated scores are based on the meta-data of the subject and the extracted features from the recorded ECG data (after exercise). Then, the CVS model is used to generate several cardiac parameters, including EF, BP, etc. After that, using those parameters and the previously evaluated features, the scores are estimated. Results show that the model-based cardiac scores better correlate with the weight of the subjects, so, it could be utilized as a clinical decision support system for physicians.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs, GPUs etc.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor-implemented method (200) comprising:

   receiving (202), via an input/output interface, a meta-data information of a subject, wherein the meta-data of the subject comprising height, weight, and age of the subject;
   collecting (204), via one or more hardware processors, a wearable electrocardiogram (ECG) data of the subject, wherein the subject is being instructed to:
   complete a predefined physical activity for a predefined period; sit up straight after completing the predefined physical activity for a predefined period to collect the wearable ECG data; and remain stable with no movement during the data collection;
   estimating (206), via the one or more hardware processors, one or more cardiac parameters in each cardiac cycle of the wearable ECG data associated with the subject using a cardiovascular simulation (CVS) model, wherein the one or more cardiac parameters include an ejection fraction (EF), an arterial blood pressure (BP), cardiac output (CO) and
   a continuous heart rate (HR);
   calculating (208), via the one or more hardware processors, a body-metabolic index (BMI) of the subject using a standard scoring method and a normal BMI range;
   estimating (210), via the one or more hardware processors, a meta score by combining the received meta-data information, the estimated one or more cardiac parameters in each cardiac cycle of the wearable ECG data and the calculated BMI of the subject; and
   determining (212), via the one or more hardware processors, the cardiac score in a recovery period post-exercise from the estimated meta score based on a predefined normalized scale.

2. The processor-implemented method (200) as claimed in claim 1, wherein the CVS model comprising a pair of atriums and ventricles functioning as a pulsatile pump.

3. The processor-implemented method (200) as claimed in claim 1, wherein the continuous heart rate and one or more cardiac compliance parameters are estimated using the wearable ECG data.

4. The processor-implemented method (200) as claimed in claim 1, wherein pressures-volumes are updated across the cardiac chambers based on the estimated continuous heart rate and one or more cardiac compliance parameters.

5. The processor-implemented method (200) as claimed in claim 1, wherein the meta-data information is utilized to define a total blood volume and an unstressed blood volume of the subject.

6. The processor-implemented method (200) as claimed in claim 5, wherein the unstressed blood volume of the subject is an auto-regulated by a baroreflex autoregulation principle.

7. A system (100) comprising:

   an input/output interface (104) to receive a meta-data information of a subject, wherein the meta-data of the subject comprising height, weight, and age of the subject;
   a memory (110) in communication with the one or more hardware processors (108), wherein the one or more hardware processors (108) are configured to execute programmed instructions stored in the memory (110) to;

      collect a wearable electrocardiogram (ECG) data of the subject,
      wherein the subject is being instructed to:

         complete a predefined physical activity for a predefined period;
         sit up straight after completing the predefined physical activity for a predefined period to collect the wearable ECG data; and
         remain stable with no movement during the data collection;

      estimate one or more cardiac parameters in each cardiac cycle of the wearable ECG data associated with the subject using a cardiovascular simulation (CVS) model, wherein the one or more cardiac parameters include an ejection fraction (EF), an arterial blood pressure (BP), cardiac output (CO) and a continuous heart rate (HR);

calculate a body-metabolic index (BMI) of the subject using a standard scoring method and a normal BMI range;

estimate a meta score by combining the received meta-data information, the estimated one or more cardiac parameters in each cardiac cycle of the wearable ECG data and the calculated BMI of the subject; and

determine the cardiac score in a recovery period post-exercise from the estimated meta score based on a predefined normalized scale.

8. The system (100) as claimed in claim 7, wherein the CVS model comprising a pair of atriums and ventricles functioning as a pulsatile pump.

9. The system (100) as claimed in claim 7, wherein the continuous heart rate and one or more cardiac compliance parameters are estimated using the wearable ECG data.

10. The system (100) as claimed in claim 7, wherein pressures-volumes are updated across the cardiac chambers based on the estimated continuous heart rate and one or more cardiac compliance parameters.

11. The system (100) as claimed in claim 7, wherein the meta-data information is utilized to define a total blood volume and an unstressed blood volume of the subject.

12. The system (100) as claimed in claim 11, wherein the unstressed blood volume of the subject is auto regulated by a baroreflex autoregulation principle.

13. One or more non-transitory machine-readable information storage mediums comprising one or more instructions which when executed by one or more hardware processors cause:

receiving, via an input/output interface, a meta-data information of a subject, wherein the meta-data of the subject comprising height, weight, and age of the subject;

collecting a wearable electrocardiogram (ECG) data of the subject,

wherein the subject is being instructed to:

complete a predefined physical activity for a predefined period; sit up straight after completing the predefined physical activity for a predefined period to collect the wearable ECG data; and remain stable with no movement during the data collection;

estimating one or more cardiac parameters in each cardiac cycle of the wearable ECG data associated with the subject using a cardiovascular simulation (CVS) model, wherein the one or more cardiac parameters include an ejection fraction (EF), an arterial blood pressure (BP), cardiac output (CO) and a continuous heart rate (HR) and wherein the CVS model comprises a pair of atriums and ventricles functioning as a pulsatile pump;

calculating a body-metabolic index (BMI) of the subject using a standard scoring method and a normal BMI range;

estimating a meta score by combining the received meta-data information, the estimated one or more cardiac parameters in each cardiac cycle of the wearable ECG data and the calculated BMI of the subject; and

determining the cardiac score in a recovery period post-exercise from the estimated meta score based on a predefined normalized scale.

14. The one or more non-transitory machine-readable information storage mediums as claimed in claim 13, wherein the continuous heart rate and one or more cardiac compliance parameters are estimated using the wearable ECG data and wherein pressures-volumes are updated across the cardiac chambers based on the estimated continuous heart rate and one or more cardiac compliance parameters.

15. The one or more non-transitory machine-readable information storage mediums as claimed in claim 13, wherein the meta-data information is utilized to define a total blood volume and an unstressed blood volume of the subject, and wherein the unstressed blood volume of the subject is an auto-regulated by a baroreflex autoregulation principle.

100

104-1

106

104-2

104-N

102

108

110

112

FIG. 1

200

Receiving a meta-data information of a subject, wherein the meta-data of the subject comprising height, weight, and age of the subject —— 202

Collecting wearable electrocardiogram (ECG) data of the subject —— 204

Estimating one or more cardiac parameters in each cardiac cycle of the wearable ECG data associated with the subject using a cardiovascular simulation (CVS) model —— 206

Calculating a body-metabolic index (BMI) of the subject using a standard scoring method and a normal BMI range —— 208

Estimating a meta score by combining the received meta-data information, the estimated one or more cardiac parameters in each cardiac cycle of the wearable ECG data and the calculated BMI of the subject —— 210

Determining the cardiac score in a recovery period post-exercise from the estimated meta score based on a predefined normalized scale —— 212

FIG. 2

300

Estimated features from the
wearable ECG Data

Height, Weight, and Age

Cardiac recovery score
without model

Cardiac recovery score with
model outputs

Cardiac parameters

CVS Model

Unstressed volume estimation
through baroreflex

FIG. 3

400

FIG. 4A

400

**FIG. 4B**

**EP 4 447 068 A1**

Europäisches Patentamt
European Patent Office
Office européen des brevets

# EUROPEAN SEARCH REPORT

Application Number

EP 24 17 0013

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/384044 A1 (ULLOA-CERNA ALVARO E [US] ET AL) 1 December 2022 (2022-12-01)<br>* abstract *<br>* paragraph [0006] - paragraph [0008] *<br>* paragraph [0032] - paragraph [0105] *<br>* claims 1-11,29,30 * | 1-15 | INV.<br>G16H50/30<br>A61B5/029<br>A61B5/00 |
| X | US 2015/164349 A1 (GOPALAKRISHNAN RAVI [US] ET AL) 18 June 2015 (2015-06-18)<br>* abstract *<br>* paragraph [0009] - paragraph [0022] *<br>* paragraph [0043] - paragraph [0093] *<br>* claims 21-25,35,37 *<br>* figure 7 * | 1-15 | |
| A | US 2015/126822 A1 (CHAVAN ABHI [US] ET AL) 7 May 2015 (2015-05-07)<br>* abstract *<br>* claims 1,4 * | 1-15 | |
| A | ROY DIBYENDU ROY DIBYENDU@TCS COM ET AL: "Wearable sensor driven Cardiac model to derive hemodynamic insights during exercise",<br>PROCEEDINGS OF THE 2021 ACM SIGIR INTERNATIONAL CONFERENCE ON THEORY OF INFORMATION RETRIEVAL, ACMPUB27, NEW YORK, NY, USA,<br>24 June 2021 (2021-06-24), pages 30-35, XP058767847,<br>DOI: 10.1145/3469260.3469670<br>ISBN: 978-1-4503-8620-3<br>* the whole document * | 1-15 | **TECHNICAL FIELDS SEARCHED (IPC)**<br><br>G16H<br>A61B |

The present search report has been drawn up for all claims

| Place of search | Date of completion of the search | Examiner |
|---|---|---|
| Munich | 16 August 2024 | Hilbig, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 0013

This annex lists the patent family members relating to the patent documents cited in the above-mentioned European search report.
The members are as contained in the European Patent Office EDP file on
The European Patent Office is in no way liable for these particulars which are merely given for the purpose of information.

16-08-2024

| Patent document cited in search report | Publication date | Patent family member(s) | Publication date |
|---|---|---|---|
| US 2022384044 A1 | 01-12-2022 | AU 2022280964 A1 | 21-12-2023 |
| | | AU 2022281440 A1 | 14-12-2023 |
| | | BR 112023024902 A2 | 20-02-2024 |
| | | CA 3220416 A1 | 10-12-2022 |
| | | CA 3220417 A1 | 01-12-2022 |
| | | EP 4346564 A1 | 10-04-2024 |
| | | EP 4346565 A1 | 10-04-2024 |
| | | EP 4346566 A1 | 10-04-2024 |
| | | JP 2024522121 A | 11-06-2024 |
| | | US 2022384044 A1 | 01-12-2022 |
| | | US 2022384045 A1 | 01-12-2022 |
| | | US 2023343464 A1 | 26-10-2023 |
| | | WO 2022251747 A1 | 01-12-2022 |
| | | WO 2022251748 A1 | 01-12-2022 |
| | | WO 2022251750 A1 | 01-12-2022 |
| US 2015164349 A1 | 18-06-2015 | EP 3079571 A1 | 19-10-2016 |
| | | US 2015164349 A1 | 18-06-2015 |
| | | US 2015265164 A1 | 24-09-2015 |
| | | US 2017238814 A1 | 24-08-2017 |
| | | US 2019038149 A1 | 07-02-2019 |
| | | US 2020022594 A1 | 23-01-2020 |
| | | US 2020229713 A1 | 23-07-2020 |
| | | US 2023099854 A1 | 30-03-2023 |
| | | WO 2015089484 A1 | 18-06-2015 |
| US 2015126822 A1 | 07-05-2015 | CN 105873504 A | 17-08-2016 |
| | | EP 3065635 A1 | 14-09-2016 |
| | | JP 6455843 B2 | 23-01-2019 |
| | | JP 2017500076 A | 05-01-2017 |
| | | US 2015126822 A1 | 07-05-2015 |
| | | WO 2015066430 A1 | 07-05-2015 |

EPO FORM P0459

For more details about this annex : see Official Journal of the European Patent Office, No. 12/82

**REFERENCES CITED IN THE DESCRIPTION**

**Patent documents cited in the description**

- IN 202321017923